# EUROPEAN PATENT APPLICATION

(11) **EP 1 816 136 A1**
(43) Date of publication of application: **08.08.2007**
(21) Application number: 05807057.4
(22) Date of filing: 17.11.2005
(51) Int. Cl.: C07K 7/02, A61K 38/00, A61P 3/04, A61P 3/10, A61P 5/06, A61P 25/06, A61P 25/18, A61P 25/20, A61P 29/00, G01N 33/15, G01N 33/50

(54) **GPR7-SELECTIVE LIGAND AND USE THEREOF**

(30) Priority: 19.11.2004 JP 2004336450
(71) Applicant: BANYU PHARMACEUTICAL CO., LTD., Tokyo 102-8667 (JP)
(72) Inventor: MATSUDA, Masao, c/o Banyu Pharmaceutical Co.,Ltd., Tsukuba-shi, Ibaraki 3002611 (JP); TOKITA, Shigeru, c/o Banyu Pharmaceutical Co.,Ltd., Tsukuba-shi, Ibaraki 3002611 (JP); KANATANI, Akio, c/o Banyu Pharmaceutical Co.,Ltd., Tsukuba-shi, Ibaraki 3002611 (JP); KANESAKA, Maki, c/o Banyu Pharmaceutical Co.,Ltd., Tsukuba-shi, Ibaraki 3002611 (JP)
(74) Representative: Buchan, Gavin MacNicol
(86) International application number: PCT/JP2005/021121
(87) International publication number: WO 2006/054641

(57) **Abstract**

A compound represented by the following formula, or a pharmaceutically acceptable salt thereof is a ligand selective for GPR7:
Trp-Tyr-Lys-(X)ₙ-Gly-Arg-Ala-Ala-Gly-Leu-Leu-Ser-
Gly-Leu-NH₂
where n is an integer of 2 to 10, and X independently represents a group represented by -NH-(CH₂)ₘ-CO- (where m is an integer of 2 to 6), a group represented by the formula 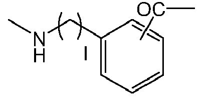 (where 1 is 0 or 1) which may have a substituent, or the like.

## Description

### Technical Field

This invention relates to a ligand selective for GPR7, and its use.

### Background Art

Neuropeptide W (NPW) and neuropeptide B (NPB) are reported as intrinsic ligands for GPR7 and GPR8 which are orphan receptors. These peptides are suggested to be involved in food intake, energy metabolism and endocrine regulation (Patent Document 1 and Non-patent Documents 1 to 4).

The in vivo localization of GPR7 and that of GPR8 are different, and GPR7 and GPR8 are presumed to have their own physiological functions. Thus, ligands with high selectivity for the respective receptors are very useful in investigating the functions of these receptors.
Patent Document 1: Japanese Unexamined Patent Publication No. 2004-49003
Non-patent Document 1: Mondal MS. et al., Endocrinology, vol. 144, pp. 4729-4733 (2003)
Non-patent Document 2: Tanaka H. et al., Proceedings of the National Academy of Sciences of the United States of America, vol. 100, pp. 6251-6256 (2003)
Non-patent Document 3: Fujii R. et al., The Journal of Biological Chemistry, vol. 277, pp. 34010-34016 (2002)
Non-patent Document 4: Shimomura Y. et al., The Journal of Biological Chemistry, vol. 277, pp. 35826-35832 (2002)
Non-patent Document 5: Winsky-Sommerer R. et al., SLEEP, vol. 26, Abstract Supplement, 0118.A (2003)

### Disclosure of the Invention

### Problems to be Solved by the Invention

However, NPW and NPB as intrinsic ligands do not have high selectivity for GPR7. A ligand with high selectivity for GPR7 is considered to be a useful tool for elucidating the functions of GPR7.

Moreover, GPR7 and the ligand for GPR7 are known to be related to various diseases and symptoms. Examples of their relations are: (1) relation to obesity, diabetes, and eating disorder, and relation to insufficiency in secretion of pituitary hormones (Patent Document 1); (2) effect on sleep and arousal (Non-patent Document 5); (3) effect on pain sensation (Non-patent Document 2); and (4) effect on stress (Non-patent Document 2). Hence, the ligand for GPR7 can serve as a drug for prevention and/or treatment of these diseases.

Furthermore, the ligand with high selectivity for GPR7 can be applied in screening pharmaceutical candidate compounds for preventive and/or therapeutic agents for the above-mentioned diseases.

Therefore, it is an object of the present invention to provide a ligand selective for GPR7 and its uses.

### Means for Solving the Problems

To attain the above object, the inventors synthesized various analogues of NPB, and found compounds with very high selectivity for GPR7. Based on this finding, they have accomplished the present invention.

That is, the present invention provides a compound represented by the following formula: Trp-Tyr-Lys-(X)ₙ-Gly-Arg-Ala-Ala-Gly-Leu-Leu-Ser-Gly-Leu-NH₂ or a pharmaceutically acceptable salt thereof:
wherein
n is an integer of 2 to 10,
X independently represents
a group represented by -NH-(CH₂)ₘ-CO- (where m is an integer of 2 to 6),
a group represented by the formula: 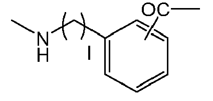 which may have a substituent selected from a substituent group A,
wherein 1 is 0 or 1,
a group represented by the formula: 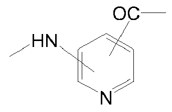 which may have a substituent selected from the substituent group A,
a group represented by the formula: 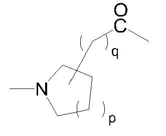 which may have a substituent selected from the substituent group A,
where p is an integer of 0 to 2, and q is 0 or 1, or
a group represented by the formula: 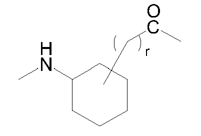 which may have a substituent selected from the substituent group A,
wherein r is 0 or 1;
and the substituent group A consists of a halogen, a methyl group, an ethyl group, a hydroxyl group, a methoxy group, and a nitro group.

Here, X is preferably a group represented by -NH-(CH₂)₄-CO-. In this case, n is preferably an integer of 2 to 5, especially 3.

It is also preferred that n be 3, two of the X's be each a group represented by -NH-(CH₂)₄-CO-, and the remaining X be
a group represented by the formula: 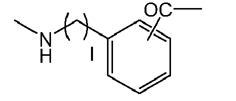 which may have a substituent selected from the substituent group A,
wherein 1 is 0 or 1,
a group represented by the formula: 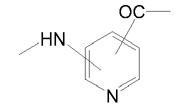 which may have a substituent selected from the substituent group A,
a group represented by the formula: 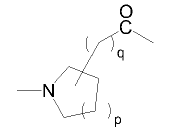 which may have a substituent selected from the substituent group A,
wherein p is an integer of 0 to 2, and q is 0 or 1, or
a group represented by the formula: 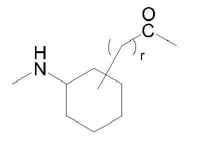 which may have a substituent selected from the substituent group A,
wherein r is 0 or 1.

It is particularly preferred that n be 3, two of the X's be each a group represented by -NH-(CH₂)₄-CO-, and the remaining X be a group represented by the formula: 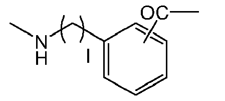 which may have a substituent selected from the group consisting of a halogen, a methyl group, an ethyl group, a hydroxyl group, a methoxy group, and a nitro group,
wherein 1 is 0 or 1.

It is further preferred that n be 3, two of the X's be each a group represented by -NH-(CH₂)₄-CO-, and the remaining X be a group represented by the formula:

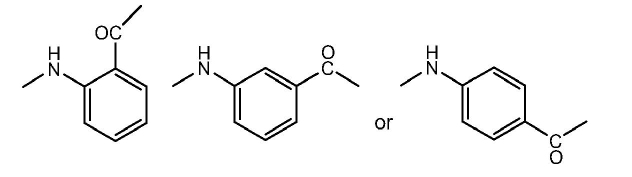

The above compound or its pharmaceutically acceptable salt is a ligand selective for GPR7, and is an agonist selective for GPR7.

The present invention also provides a drug containing the above-mentioned compound, or its pharmaceutically acceptable salt, as an active ingredient. More concretely, the present invention provides a prophylactic and/or therapeutic agent for obesity, diabetes or hyperphagia, an aperitive agent, a prophylactic and/or therapeutic agent for pituitary hormone secretion insufficiency, a prophylactic and/or therapeutic agent for sleep disorder, an analgesic, and a prophylactic and/or therapeutic agent for stress.

The present invention also provides a method for screening compounds binding to GPR7, comprising the steps of:
(a) bringing GPR7 and the compound of the present invention or its pharmaceutically acceptable salt into contact with each other under conditions where a candidate compound is present and under conditions where the candidate compound is not present, and measuring the amount of binding between GPR7 and the compound of the present invention or its pharmaceutically acceptable salt, and
(b) selecting the candidate compound which changes the amount of binding between GPR7 and the compound of the present invention or its pharmaceutically acceptable salt.

### Effects of the Invention

The compound of the present invention has very high selectivity for GPR7, and thus serves as a useful tool for elucidating the functions of GPR7. Also, the compound of the present invention becomes a prophylactic and/or therapeutic agent for various diseases including eating disorder. Furthermore, the compound of the present invention can be applied to screening of pharmaceutical candidate compounds for prophylactic and/or therapeutic agents for various diseases.

### Best Mode for Carrying Out the Invention

### Compound:

The compound of the present invention will be described below. The compound of the present invention is represented by Trp-Tyr-Lys-(X)ₙ-Gly-Arg-Ala-Ala-Gly-Leu-Leu-Ser-Gly-Leu-NH₂. This compound is an analogue of hNPB (human neuropeptide B) which has been formed by substituting amino acids, as the 4th to 13th residues of desBr-hNPB(1-23), by n groups (corresponding to X) each having an amino group and a carboxyl group. Leu-NH₂ represents that the carboxyl group of the leucine residue at the C-terminal has been amidated. Lys-(X)ₙ-Gly represents that the carboxyl group of the lysine residue (corresponding to the 3rd residue of hNPB) and the amino group of the first X (counting from the tryptophan as the N-terminal; the same applies hereinafter) are bound to form an amide bond, the amino group of the ith X and the carboxyl group of the (i+1)th X are bound to form an amide bond, and the carboxyl group of the nth X and the amino group of the glycine residue (corresponding to the 14th residue of hNPB) are bound to form an amide bond.

Here, n is an integer of 2 to 10.

X independently represents any of the following groups (1) to (5):
(1) a group represented by -NH-(CH₂)ₘ-CO- (where m is an integer of 2 to 6),
(2) a group represented by the formula: 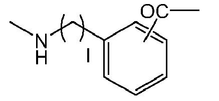 (where 1 is 0 or 1) which may have a substituent selected from a substituent group A,
(3) a group represented by the formula: 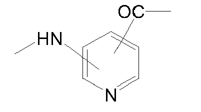 which may have a substituent selected from the substituent group A,
(4) a group represented by the formula: 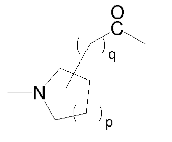 (where p is an integer of 0 to 2, and q is 0 or 1) which may have a substituent selected from the substituent group A, or
(5) a group represented by the formula: 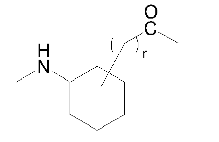 (where r is 0 or 1) which may have a substituent selected from the substituent group A.

The term "may have a substituent selected from the substituent group A" refers to the fact that any one hydrogen atom or any plural hydrogen atoms on the ring (a benzene ring, a pyridine ring, azetidine, a pyrrolidine ring, a piperidine ring, and a cyclohexane ring) may be substituted by a substituent or substituents selected from the substituent group A (a halogen, a methyl group, an ethyl group, a hydroxyl group, a methoxy group, and a nitro group).

The group represented by the formula: 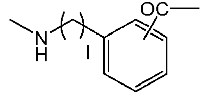 (where 1 is 0 or 1) represents a divalent group having any hydrogen atoms of a benzene ring substituted by -NH-(CH₂)₁- and -CO-, and its specific examples are groups of the following formulae:

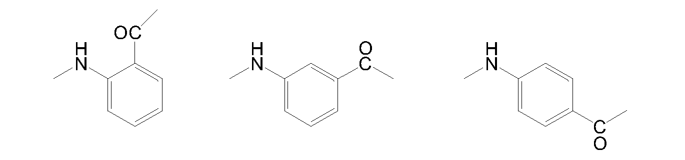

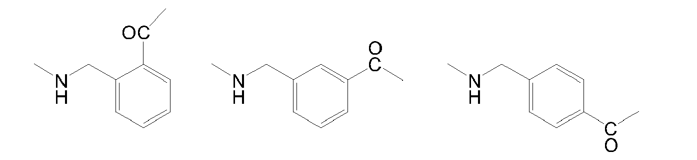

The group represented by the formula: 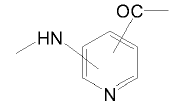 represents a divalent group having any hydrogen atoms of a pyridine ring substituted by -NH- and -CO-, and its specific examples are groups of the following formulae:

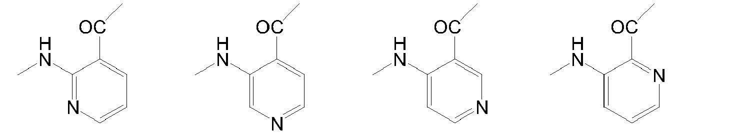

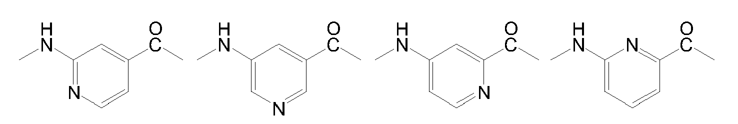

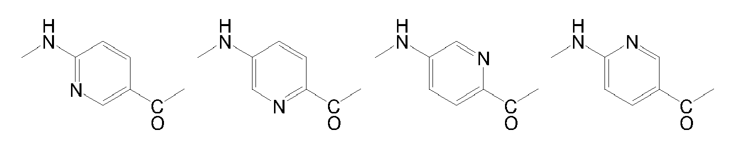

The group represented by the formula: 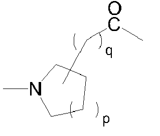 (where p is an integer of 0 to 2, and q is 0 or 1) represents a divalent group having any hydrogen atom of an azetidin-1-yl group, a pyrrolidin-1-yl group, or a piperidin-1-yl group substituted by -(CH₂)_{q}-CO-, and its specific examples are groups of the following formulae:

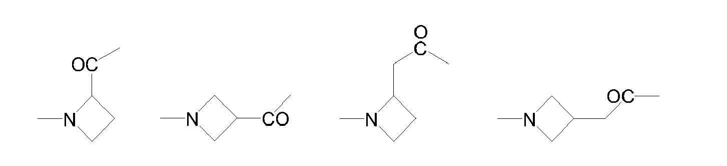

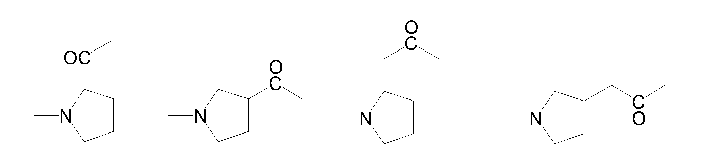

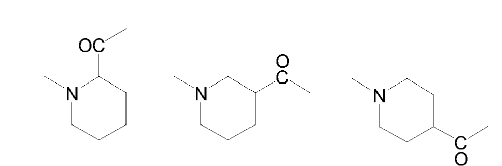

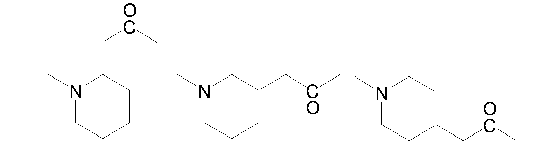

The group represented by the formula: 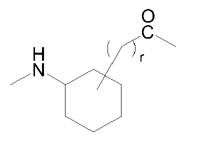 (where r is 0 or 1) represents a divalent group having any hydrogen atoms of a cyclohexane ring substituted by -NH- and -(CH₂)ᵣ-CO-, and its specific examples are groups of the following formulae:

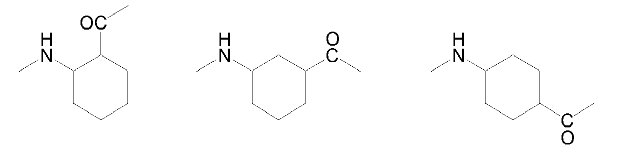

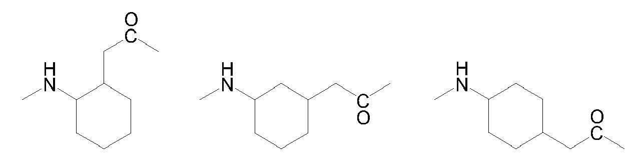

The compound of the present invention can be synthesized, for example, by applying a publicly known method of peptide synthesis. The publicly known method of peptide synthesis is, for example, a solid phase synthesis process or a liquid phase synthesis process by the Fmoc method or the Boc method. The solid phase synthesis process by the Fmoc method, in particular, is better from the points of view of yield, etc. As the amino acid or X protected with a Fmoc group used in the solid phase synthesis process by the Fmoc method, a commercially available one can be utilized. If necessary, the protected amino acid or X can be synthesized by a publicly known method. A peptide chain is elongated up to the N-terminal (tryptophan), and then the resulting peptide is treated with trifluoroacetic acid or the like to perform deprotection. The resulting compound of the present invention is cut away from the solid phase (resin), and purified (for example, reversed-phase HPLC using an ODS column or the like) if desired.

The compound of the present invention is preferably the one in which X is the group represented by -NH-(CH₂)₄-CO- (corresponding to 5-aminovaleric acid (abbreviated as Ava)). In this case, n is particularly preferably an integer of 2 to 5 and, most preferably, n is 3. If X is Ava, the compound of the present invention has moderate flexibility and a moderate chain length, and is expected to fit GPR7. If n is an integer of 2 to 5, moreover, the compound of the present invention is highly specific for GPR7. The compound of the present invention with n being 3, in particular, has selectivity for GPR7 which is about 2,900 times its selectivity for GPR8.

With the compound of the present invention, it is also preferred that n be 3, two of the X's be each a group represented by the formula -NH-(CH₂)₄-CO-, and the remaining X be
the group represented by the formula: 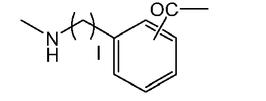 (where 1 is 0 or 1) which may have the substituent selected from the substituent group A,
the group represented by the formula: 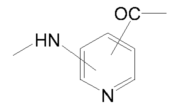 which may have the substituent selected from the substituent group A,
the group represented by the formula: 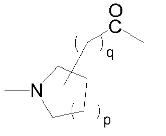 (where p is an integer of 0 to 2, and q is 0 or 1) which may have the substituent selected from the substituent group A, or
the group represented by the formula: 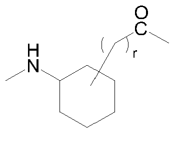 (where r is 0 or 1) which may have the substituent selected from the substituent group A.

Because of possession of two of the groups represented by the formula -NH-(CH₂)₄-CO-, the compound of the present invention has moderate flexibility and a moderate chain length. Because of having one benzene ring, one pyridine ring, one azetidine, one pyrrolidine ring, one piperidine ring, or one cyclohexane ring, the compound of the present invention has moderate rigidity, and is expected to fit GPR7.

From the viewpoint of moderate rigidity, X is preferably a benzene ring or a pyridine ring, and particularly preferably a benzene ring. That is, X is preferably the group represented by the formula: 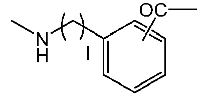 (where 1 is 0 or 1) which may have the substituent selected from the group consisting of a halogen, a methyl group, an ethyl group, a hydroxyl group, a methoxy group, and a nitro group. If, in particular, X is preferably the group represented by the formula: 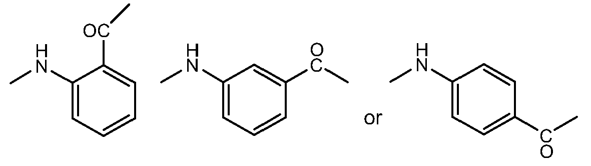 (corresponding to 2-, 3- and 4-aminobenzoic acid (abbreviated as 2-Abz, 3-Abz and 4-Abz, respectively)), the compound of the present invention has high specificity for GPR7, so that X is preferably any of these groups. If X is 2-Abz, in particular, the selectivity for GPR7 is about 1,700 times the selectivity for GPR8.

The pharmaceutically acceptable salt of the compound of the present invention is also included in the scope of the present invention. As the pharmaceutically acceptable salt, an acid-addition salt and a base-addition salt are named. Examples of the acid-addition salt are hydrohalogenic acid salts such as hydrochloride, hydrofluoride, hydrobromide, and hydriodide; inorganic acid salts such as nitrate, perchlorate, sulfate, phosphate, and carbonate; lower alkylsulfonates such as methanesulfonate, trifluoromethanesulfonate, and ethanesulfonate; arylsulfonates such as benzenesulfonate and p-toluenesulfonate; and organic acid salts such as fumarate, succinate, citrate, tartrate, oxalate, and maleate. Examples of the base-addition salts are salts of alkali metals such as sodium and potassium; salts of alkaline earth metals such as calcium and magnesium; and salts derived from organic bases such as ammonium salt and salts of guanidine, triethylamine, and dicyclohexylamine. The compound of the present invention can be converted into pharmaceutically acceptable salts by customary methods.

### GPR7-selective ligand and GPR7-selective agonist:

The GPR7-selective ligand and GPR7-selective agonist of the present invention comprise the compound of the present invention or its pharmaceutically acceptable salt. The fact that the ligand and the agonist are selective for GPR7 means that the ligand, etc. bind more strongly to GPR7 than to GPR8 (have a high binding constant), or that the EC₅₀ (50% effective concentration) of the ligand, etc. is lower for GPR7 than for GPR8.

The compound of the present invention or its pharmaceutically acceptable salt has high selectivity for GPR7. For example, as shown in the Examples (to be offered later), the results of binding inhibition experiments (comparison of IC₅₀ (50% inhibition concentration) demonstrate the compound of the present invention or its pharmaceutically acceptable salt to be about 15 to 2,900 times selective (Table 1 and Table 2). The results of the EC₅₀ measurements show the compound of the present invention or its pharmaceutically acceptable salt to be about 4 to 165 times selective (Table 1). Thus, the compound of the present invention or its pharmaceutically acceptable salt functions as a GPR7-selective ligand and a GPR7-selective agonist.

### Drug:

The drug of the present invention contains the compound of the present invention or its pharmaceutically acceptable salt as an active ingredient. The drug of the present invention can be applied to diseases and symptoms in which GPR7 and GPR7 ligand are involved. Concrete examples of the drug are prophylactic and/or therapeutic agents for obesity, diabetes or hyperphagia, aperitive agents, prophylactic and/or therapeutic agents for pituitary hormone secretion insufficiency, prophylactic and/or therapeutic agents for sleep disorder (insomnia, etc.), analgesics, and prophylactic and/or therapeutic agents for stress (stress-associated hyperphagia, insomnia, etc.).

The drug of the present invention can be formed into various pharmaceutical products by adding pharmaceutically acceptable additives to the compound of the present invention in conformity with the mode of administration of the drug. As the additives, various additives usually used in the field of pharmaceutical products can be used. Their examples are gelatin, lactose, sucrose, titanium oxide, starch, microcrystalline cellulose, hydroxypropyl methylcellulose, carboxymethylcellulose, corn starch, microcrystalline wax, white petrolatum, magnesium methasilicate aluminate, anhydrous calcium phosphate, citric acid, trisodium citrate, hydroxypropylcellulose, sorbitol, sorbitan esters of fatty acid, polysorbate, sucrose fatty acid ester, polyoxyethylene, hydrogenated castor oil, polyvinylpyrrolidone, magnesium stearate, light anhydrous silicic acid, talc, vegetable oil, benzyl alcohol, acacia, propylene glycol, polyalkylene glycol, cyclodextrin, and hydroxypropyl cyclodextrin.

Dosage forms pharmaceutically manufactured as mixtures with these additives include, for example, solid pharmaceutical products, such as tablets, capsules, granules, powders, and suppositories; and pharmaceutical products in liquid and solution forms, such as syrups, elixirs, and injections. These pharmaceutical products can be prepared in accordance with ordinary methods in the field of pharmaceutical products. The pharmaceutical products as liquids and solutions may be in forms which are dissolved or suspended in water or other suitable medium when they are used. In the case of the injections, in particular, the active principle may be dissolved or suspended in physiological saline or a glucose solution, where necessary, and buffering agents or preservatives may be further added.

If the compound of the present invention is used, for example, in the clinical setting, its dose and dosing frequency are different according to the sex, age, body weight and severity of symptoms of a patient, the type and scope of a measure taken, and the intended effect. In the case of oral administration, it is generally preferred to administer an adult daily dose of 0.01 to 100 mg/kg, preferably 0.03 to 1 mg/kg, in a single to several portions. With parenteral administration, it is generally preferred to administer an adult daily dose of 0.001 to 10 mg/kg, preferably 0.001 to 0.1 1 mg/kg, more preferably 0.01 to 0.1 1 mg/kg, in a single to several portions. An ordinary internist, veterinarian, or clinician can easily determine and handle an effective dose necessary to inhibit, suppress or stop the progress of disease.

The drug of the present invention can contain the compound of the present invention in a proportion of 1.0 to 100% by weight, preferably 1.0 to 60% by weight, based on the entire pharmaceutical. Any of the pharmaceutical products of the present invention may also contain other therapeutically effective compounds.

### Screening method:

The method of the present invention, which screens compounds binding to GPR7, comprises the following steps:
(a) bringing GPR7 and the compound of the present invention or a pharmaceutically acceptable salt thereof into contact with each other under conditions where a candidate compound is present and under conditions where a candidate compound is not present, and measuring the amount of binding between GPR7 and the compound of the present invention or the pharmaceutically acceptable salt thereof, and
(b) selecting the candidate compound which changes the amount of binding between GPR7 and the compound of the present invention or the pharmaceutically acceptable salt thereof.

GPR7 is related to various diseases and symptoms. Thus, compounds binding to GPR7 (i.e., agonists and antagonists) can serve as prophylactic and/or therapeutic agents for diseases, etc. in which GPR7 is involved. Their concrete examples are prophylactic and/or therapeutic agents for obesity, diabetes or hyperphagia, aperitive agents, prophylactic and/or therapeutic agents for pituitary hormone secretion insufficiency, prophylactic and/or therapeutic agents for sleep disorder (insomnia, etc.), analgesics, and prophylactic and/or therapeutic agents for stress (stress-associated hyperphagia, insomnia, etc.). According to the screening method of the present invention, candidate compounds for these drugs can be screened.

As GPR7 for use in the screening method of the present invention, the membrane fraction of GPR7 expression cells can be utilized. GPR7 is preferably derived from a mammal, especially a human. The membrane fraction of GPR7 expression cells can be prepared in accordance with the method described in Japanese Unexamined Patent Publication No. 2004-49003. A concrete method of preparation is as follows: Based on publicly known sequence information, cDNA of GPR7 is amplified by the PCR method or the like, and the amplified cDNA is inserted into an appropriate expression vector for cloning. The expression vector having GPR7 inserted therein is transfected into host cells to obtain GPR7 expression cells. The GPR7 expression cells are disrupted, and a membrane fraction is prepared from a cell disruption mixture by fractional centrifugation or density-gradient centrifugation. The membrane fraction contains expressed GPR7 and membrane components, such as phospholipids and membrane proteins, in large amounts. A person skilled in the art can easily perform the preparation of the membrane fraction of GPR7 expression cells by use of a publicly known method. GPR7, which is used in the screening method of the present invention, need not be the membrane fraction of GPR7 expression cells, but may be the GPR7 expression cells per se.

The compound of the present invention used in the screening method of the present invention is preferably labeled in order to measure the amount of its binding to GPR7. The label and the labeling method are not limited, and a label and a labeling method publicly known among people skilled in the art can be utilized. Concretely, radiolabeling with [³H], [¹²⁵I] [¹⁴C] [³⁵S], etc. can be utilized, and radiolabeling with [¹²⁵I] is preferred.

The step (a) is the step of bringing GPR7 and the compound of the present invention into contact with each other under conditions where a candidate compound is present and under conditions where the candidate compound is absent, and measuring the amount of their binding. Here, the contact between GPR7 and the compound of the present invention is performed in a suitable buffer. Preferably, a phosphate buffer at pH 6 to 8, a TRIS-hydrochloride buffer, or the like is used, but the type of the buffer is not limited, unless the buffer inhibits the binding of GPR7, the compound of the present invention, and the candidate compound. Where necessary, a surface active agent or the like may be added to the buffer in order to decrease nonspecific binding of GPR7, the compound of the present invention, and the candidate compound. If necessary, moreover, a protease inhibitor may be added to the buffer in order to suppress the decomposition of GPR7, the compound of the present invention, and the candidate compound. Under conditions where the candidate compound is absent or present (for example, in the concentration range of 10⁻¹⁰M to 10-7M), the buffer containing a certain amount of the compound of the present invention (set, for example, in the range of 5,000 cpm to 500,000 cpm) which has been labeled, and GPR7 is incubated. The incubation temperature is preferably 4 to 37°C, and the incubation time is preferably 30 minutes to 3 hours. After the incubation, the system is filtered through a glass filter or the like, and washed with a proper amount of the buffer. Then, the amount of the label on the glass filter is measured (radioactivity is measured with a liquid scintillation counter or a γ-counter) to determine the amount of binding between GPR7 and the compound of the present invention.

The step (b) is the step of selecting the candidate compound which changes the amount of binding between GPR7 and the compound of the present invention. It is examined whether the amount of binding measured in the step (a) does not change in presence and absence of the candidate compound. For example, the candidate compound such that when a value (B₀ - NSB) obtained by subtracting the amount of nonspecific binding (NSB) from the amount of binding in the absence of the candidate compound (this amount: B₀) is taken as 100%, the amount of specific binding (i.e., B - NSB) is 50% or less can be selected as a compound binding to GPR7. Alternatively, the candidate compound having an IC₅₀ of a predetermined value (for example, 1 to 100 nM) or less may be selected as a compound binding to GPR7.

### Examples

The present invention will now be described in further detail by the following Examples, which in no way limit the invention.

### Synthesis Example 1: Synthesis of hNPW: Trp-Tyr-Lys-His-Val-Ala-Ser-Pro-Arg-Tyr-His-Thr-Val-Gly-Arg-Ala-Ala-Gly-Leu-Leu-Met-Gly-Leu-Arg-Arg-Ser-Pro-Tyr-Leu-Trp

Peptide synthesis was performed by the Fmoc method using HATU (O-(7-azabenzotriazol-1-yl)-N,N,N',N'-tetramethyluronium hexafluorophosphate) as a condensation reagent by means of the peptide synthesizer Pioneer (trademark) Model 9010 with the use of Fmoc-Trp(Boc)-NovaSyn (trademark) TGA resin (produced by Novabiochem) as the starting material. Concretely, condensation was performed in the following order: Fmoc-Leu, Fmoc-Tyr(tBu), Fmoc-Pro, Fmoc-Ser(tBu), Fmoc-Arg(Pbf), Fmoc-Arg(Pbf), Fmoc-Leu, Fmoc-Gly, Fmoc-Met, Fmoc-Leu, Fmoc-Leu, Fmoc-Gly, Fmoc-Ala, Fmoc-Ala, Fmoc-Arg(Pbf), Fmoc-Gly, Fmoc-Val, Fmoc-Thr(tBu), Fmoc-His(Trt), Fmoc-Tyr(tBu), Fmoc-Arg(Pbf), Fmoc-Pro, Fmoc-Ser(tBu), Fmoc-Ala, Fmoc-Val, Fmoc-His(Trt), Fmoc-Lys(Boc), Fmoc-Tyr(tBu), and Fmoc-Trp(Boc). As a result, Trp(Boc)-Tyr(tBu)-Lys(Boc)-His(Trt)-Val-Ala-Ser(tBu)-Pro-Arg(Pbf)-Tyr(tBu)-His(Trt)-Thr(tBu)-Val-Gly-Arg(Pbf)-Ala-Ala-Gly-Leu-Leu-Met-Gly-Leu-Arg(Pbf)-Arg(Pbf)-Ser-Pro-Tyr(tBu)-Leu-Trp(Boc)-NovaSyn (trademark) TGA resin was obtained. TFA/thioanisole/ethanedithiol/m-crosol (95/2.5/1.5/1) was added to this resin, and the mixture was shaken for 1 hour and 30 minutes at room temperature, followed by filtering off the resin. Cooled ether was added to the filtrate to obtain crude Trp-Tyr-Lys-His-Val-Ala-Ser-Pro-Arg-Tyr-His-Thr-Val-Gly-Arg-Ala-Ala-Gly-Leu-Leu-Met-Gly-Leu-Arg-Arg-Ser-Pro-Tyr-Leu-Trp as a precipitate. This crude peptide was purified by reversed-phase fractional HPLC to collect a fraction containing the desired product. The fraction was lyophilized to obtain the desired product.

The conditions for the HPLC were as follows:
Solution A: Water containing 0.1% TFA
Solution B: Acetonitrile containing 0.1 % TFA
Gradient elution: Elution for 1 minute with A/B = 95/5, followed by elution for 15 minutes with A/B = 75/25 to 60/40 (linear)
Flow velocity: 10 mL/min

### Synthesis Example 2: Synthesis of desBr-hNPB: Trp-Tyr-Lys-Pro-Ala-Ala-Gly-His-Ser-Ser-Tyr-SerVal-Gly-Arg-Ala-Ala-Gly-Leu-Leu-Ser-Gly-Leu-Arg-Arg-Ser-Pro-Tyr-Ala

Using Fmoc-Ala-NovaSyn (trademark) TGA resin as the starting material, desBr-hNPB was synthesized by the same method as that in Synthesis Example 1. Gradient elution of HPLC was performed under the following conditions: elution for 1 minute with A/B = 95/5, followed by elution for 15 minutes with A/B = 80/20 to 65/35 (linear).

### Synthesis Example 3: Synthesis of [Phe2,28]-hNPW: Trp-Phe-Lys-His-Val-Ala-Ser-Pro-Arg-Tyr-His-Thr-Val-Gly-Arg-Ala-Ala-Gly-Leu-Leu-Met-Gly-Leu-Arg-Arg-Ser-Pro-Phe-Leu-Trp

In the same manner as in Synthesis Example 1, [Phe2,28]-NPW was synthesized. Gradient elution of reversed-phase fractional HPLC was performed under the following conditions: elution for 1 minute with A/B = 95/5, followed by elution for 15 minutes with A/B = 70/30 to 55/45.

### Synthesis Example 4: Synthesis of desBr-hNPB(1-23)-NH₂: Trp-Tyr-Lys-Pro-Ala-Ala-Gly-His-Ser-Ser-Tyr-Ser-Val-Gly-Arg-Ala-Ala-Gly-Leu-Leu-Ser-Gly-Leu-NH₂

Using NovaSyn (trademark) TGA resin as the starting material, desBr-hNPB(1-23)-NH₂ was synthesized by the same method as that in Synthesis Example 1. Gradient elution of reversed phase fractional HPLC was performed under the following conditions: elution for 1 minute with A/B = 95/5, followed by elution for 10 minutes with A/B = 78/22 to 58/42 (linear).

### Synthesis Example 5: Synthesis of [des4-13,Aval]-desBr-hNPB(1-23)-NH₂: Trp-Tyr-Lys-Ava-Gly-Arg-Ala-Ala-Gly-Leu-Leu-Ser-Gly-Leu-NH₂

In the same manner as in Synthesis Example 4, [des4-13,Ava1]-desBr-hNPB(1-23)-NH₂ was synthesized. Gradient elution of reversed phase fractional HPLC was performed under the following conditions: elution for 2 minutes with A/B = 95/5, followed by elution for 15 minutes with A/B = 80/20 to 60/40 (linear).

### Synthesis Example 6: Synthesis of [des4-13,Ava2]-desBr-hNPB(1-23)-NH₂: Trp-Tyr-Lys-Ava-Ava-Gly-Arg-Ala-Ala-Gly-Leu-Leu-Ser-Gly-Leu-NH₂

In the same manner as in Synthesis Example 5, [des4-13,Ava2]-desBr-hNPB(1-23)-NH₂ was synthesized.

### Synthesis Example 7: Synthesis of [des4-13,Ava3]-desBr-hNPB(1-23)-NH₂: Trp-Tyr-Lys-Ava-Ava-Ava-Gly-Arg-Ala-Ala-Gly-Leu-Leu-Ser-Gly-Leu-NH2

In the same manner as in Synthesis Example 5, [des4-l3,Ava3]-desBr-hNPB(1-23)-NH₂ was synthesized.

### Synthesis Example 8: Synthesis of [des4-13,Ava4]-desBr-hNPB(1-23)-NH₂: Trp-Tyr-Lys-Ava-Ava-Ava-Ava-Gly-Arg-Ala-Ala-Gly-Leu-Leu-Ser-Gly-Leu-NH₂

In the same manner as in Synthesis Example 5, [des4-13,Ava4]-desBr-hNPB(1-23)-NH₂ was synthesized.

### Synthesis Example 9: Synthesis of [des4-13,Ava5]-desBr-hNPB(1-23)-NH₂: Trp-Tyr-Lys-Ava-Ava-Ava-Ava-Ava-Gly-Arg-Ala-Ala-Gly-Leu-Leu-Ser-Gly-Leu-NH₂

In the same manner as in Synthesis Example 5, [des4-13,Ava5]-desBr-hNPB(1-23)-NH₂ was synthesized.

### Synthesis Example 10: Synthesis of [des4-13,Ava-Ava-2Abz]-desBr-hNPB(1-23)-NH₂: Trp-Tyr-Lys-Ava-Ava-2Abz-Gly-Arg-Ala-Ala-Gly-Leu-Leu-Ser-Gly-Leu-NH₂

In the same manner as in Synthesis Example 5, [des4-l3,Ava-Ava-2Abz]-desBr-hNPB(1-23)-NH₂ was synthesized. Purification of the crude peptide was performed by reversed-phase fractional HPLC using Combi HT SB-C18,S-5,120A column (21.1x150 mm).

The conditions for the HPLC were as follows:
Solution A: Water containing 0.1% TFA
Solution B: Acetonitrile containing 0.1 % TFA
Gradient elution: Elution for 1 minute with A/B = 95/5, followed by elution for 8 minutes with A/B = 95/5 to 40/60 (linear)
Flow velocity: 20 mL/min

### Synthesis Example 11: Synthesis of [des4-l3,Ava-Ava-3Abz]-desBr-hNPB(1-23)-NH₂: Trp-Tyr-Lys-Ava-Ava-3Abz-Gly-Arg-Ala-Ala-Gly-Leu-Leu-Ser-Gly-Leu-NH₂

In the same manner as in Synthesis Example 10, [des4-13,Ava-Ava-3Abz]-desBr-hNPB(1-23)-NH2 was synthesized.

### Synthesis Example 12: Synthesis of [des4-13,A va-Ava-4Abz]-desBr-hNPB(1-23)-NH₂: Trp-Tyr-Lys-Ava-Ava-4Abz-Gly-Arg-Ala-Ala-Gly-Leu-Leu-Ser-Gly-Leu-NH₂

In the same manner as in Synthesis Example 10, [des4-13,Ava-Ava-4Abz]-desBr-hNPB(1-23)-NH2 was synthesized.

### Synthesis Example 13: Synthesis of [des4-13,Ava-2Abz-Ava]-desBr-hNPB(1-23)-NH₂: Trp-Tyr-Lys-Ava-2Abz-Ava-Gly-Arg-Ala-Ala-Gly-Leu-Leu-Ser-Gly-Leu-NH₂

In the same manner as in Synthesis Example 10, [des4-13,Ava-2Abz-Ava]-desBr-hNPB(1-23)-NH2 was synthesized.

### Synthesis Example 14: Synthesis of [des4-13,Ava-3Abz-Ava]-desBr-hNPB(1-23)-NH₂: Trp-Tyr-Lys-Ava-3Abz-Ava-Gly-Arg-Ala-Ala-Gly-Leu-Leu-Ser-Gly-Leu-NH₂

In the same manner as in Synthesis Example 10, [des4-13,Ava-3Abz-Ava]-desBr-hNPB(1-23)-NH2 was synthesized.

### Synthesis Example 15: Synthesis of [des4-13,Ava-4Abz-Ava]-desBr-hNPB(1-23)-NH₂: Trp-Tyr-Lys-Ava-4Abz-Ava-Gly-Arg-Ala-Ala-Gly-Leu-Leu-Ser-Gly-Leu-NH₂

In the same manner as in Synthesis Example 10, [des4-13,Ava-4Abz-Ava]-desBr-hNPB(1-23)-NH2 was synthesized.

### Synthesis Example 16: Synthesis of [des4-13,2Abz-Ava-Ava]-desBr-hNPB(1-23)-NH₂: Trp-Tyr-Lys-2Abz-Ava-Ava-Gly-Arg-Ala-Ala-Gly-Leu- Leu-Ser-Gly-Leu-NH₂

In the same manner as in Synthesis Example 10, [des4-13,2Abz-Ava-Ava]-desBr-hNPB(1-23)-NH2 was synthesized.

### Synthesis Example 17: Synthesis of [des4-13,3Abz-Ava-Ava]-desBr-hNPB(1-23)-NH2: Trp-Tyr-Lys-3Abz-Ava-Ava-Gly-Arg-Ala-Ala-Gly-Leu- Leu-Ser-Gly-Leu-NH₂

In the same manner as in Synthesis Example 10, [des4-13,3Abz-Ava-Ava]-desBr-hNPB(1-23)-NH2 was synthesized.

### Synthesis Example 18: Synthesis of [des4-13,4Abz-Ava-Ava]-desBr-hNPB(1-23)-NH2: Trp-Tyr-Lys-4Abz-Ava-Ava-Gly-Arg-Ala-Ala-Gly-Leu- Leu-Ser-Gly-Leu-NH₂

In the same manner as in Synthesis Example 10, [des4-13,4Abz-Ava-Ava]-desBr-hNPB(1-23)-NH2 was synthesized.

### Synthesis Example 19: Synthesis of [Phe2]-hNPW(1-23): Trp-Phe-Lys-His-Val-Ala-Ser-Pro-Arg-Tyr-His-Thr-Val-Gly-Arg-Ala-Ala-Gly-Leu-Leu-Met-Gly-Leu

Using Fmoc-Leu-NovaSyn (trademark) TGA resin (produced by novabuochem) as the starting material, [Phe2]-NPW(1-23) was synthesized by the same method as that in Synthesis Example 1. Gradient elution of HPLC was performed under the following conditions: elution for 2 minutes with A/B = 95/5, followed by elution for 15 minutes with A/B = 80/20 to 60/40 (linear).

### Test Example 1

### (1) Preparation of radioiodine-labeled hNPW ([¹²⁵I]-[phe2]-hNPW(1-23)) using the lactoperoxidase method

One nmol of [Phe2]-hNPW(1-23) (prepared in Synthesis Example 19) was dissolved in 5 µL of anhydrous DMSO, and the solution was mixed with 0.1M nickel chloride. Then, the mixture was mixed with 10 µL of a 0.00 1 % aqueous solution of hydrogen peroxide, 10 µL of an enzyme solution (lactoperoxidase (Sigma) adjusted to 10 µg/mL with a 0.1M HEPES buffer solution), and 37 Mbq/10 µL of Iodine-125 (Amersham), whereafter the resulting mixture was reacted for 60 minutes at room temperature. The reaction was terminated with the addition of 10 µL of 1% sodium azide, and then a radiolabeled peptide fraction was collected by a PD-10 gel filtration column (Amersham) to obtain [¹²⁵I]-[Phe2]-hNPW(1-23).

### (2) Binding inhibition test

A cDNA sequence coding for human GPR7 or GPR8 [see Genomics, vol. 28, p. 84 (1995)] was amplified by the PCR method, and cloned using the expression vector pIRES-hygro (Clontech). The resulting expression vector was transfected into HEK293/CRE-BLA cells (AURORA) using the cationic lipid method [see Proceedings of the National Academy of Sciences of the United States of America, vol. 84, p. 7413 (1987)] to obtain GPR7 or GPR8 expression cells.

A membrane preparation prepared from the cells expressing GPR7 or GPR8 was incubated for 1 hour at room temperature in an assay buffer solution (25 mM Tris buffer solution, 150 mM NaCl, pH 7.4) together with a test compound and 28,000 cpm of [¹²⁵I]-[Phe2]-hNPW(1-23). Then, the system was filtered through the glass filter GF/C. After washing with 25 mM Tris buffer solution (pH 7.4), radioactivity on the glass filter was measured. Nonspecific binding was measured in the presence of 1 µM hNPW (prepared in Synthesis Example 1), and the 50% inhibition concentration (IC₅₀ value) of the test compound against [Phe2]-hNPW(1-23) specific binding was calculated [see Molecular Pharmacology, vol. 55, 1101 (1999)].

### Test Example 2 Measurement of activation capacity of various synthetic peptides on GPR7 and GPR8

In accordance with the method of Zlakarmik (see Science, 1998, vol. 279, p. 84), the activity of various synthetic peptides on GPR7 or GPR8 was measured. HEK293/CRE-BLA cells which expressed GPR7 or GPR8 (prepared in (2) of Test Example 1) were washed twice with PBS(-) buffer solution (GIBCO-BRL), and then Cell-dissociation buffer (GIBCO-BRL) was added. The system was kept at 37°C for 3 minutes in a CO₂ incubator, and a culture flask was tapped to liberate the cells from the flask. After the cells were harvested by centrifugation, the cells were suspended in an Opti-MEM culture medium (GIBCO-BRL) containing 0.1 % BSA (Sigma) to count the cells and prepare a cell suspension containing 8×10⁵ cells/mL. To 2 µL of a DMSO solution containing each test compound with a concentration of 50 times an end concentration, 50 µL of the 0.1% BSA-containing Opti-MEM medium incorporating forskolin (Sigma) so as to have an end concentration of 1 µM was added to form a reaction solution. To this reaction solution, 50 µL of the above cell suspension was added to initiate the reaction, and the system was incubated for 3 hours at 37°C in a CO₂ incubator.

Then, using a BLA assay kit (AURORA), a coloring matter-introduced buffer solution incorporating 12 µL of Solution A [1 mM CCF2-AM/anhydrous DMSO solution], 120 µL of Solution B [100 mg/mL Pluronic 127, 0.1% acetic acid/DMSO solution], and 2 mL of Solution C [24% (w/w) PEG-400, 18% TR40/H₂O] was prepared. This coloring matter-introduced buffer solution (20 µL) was added to the above-mentioned cell reaction mixture to introduce a β-lactamase substrate (CCF2-AM) into the cells. After the system was allowed to stand for 1 hour at room temperature, the fluorescence intensities of the sample at an excitation wavelength of 409 nm, at a fluorescence wavelength of 460 nm (a β-lactamase hydrolyzate of CCF), and at 530 nm (CCF) were measured. With β-lactamase activity (fluorescence intensity at 460 nm/fluorescence intensity at 530 nm) as an indicator, the activity of each of various peptides on GPR7 or GPR8 was measured, and the 50% effective concentration (EC₅₀) of the test compound was calculated.

The IC₅₀ and EC₅₀ when each of the peptides obtained in Synthesis Examples 4 to 9 was used are shown in Table 1. The IC₅₀ when each of the peptides obtained in Synthesis Examples 10 to 18 and 1 was used is shown in Table 2.

**Table 1**

| Synthesis Example | GPR7 (nM) | | GPR8 (nM) | |
|---|---|---|---|---|
| | IC₅₀ | EC₅₀ | IC₅₀ | EC ₅₀ |
| 4 | 0.23 | 0.24 | 24 | 0.86 |
| 5 | 0.55 | 1.7 | 310 | 280 |
| 6 | 0.47 | 2.2 | 300 | 94 |
| 7 | 0.09 | 0.48 | 260 | 77 |
| 8 | 1.1 | 1.2 | 360 | 51 |
| 9 | >100 | not tested | 2600 | not tested |

**Table 2**

| Synthesis Example | GPR7 (nM) | GPR8 (nM) |
|---|---|---|
| 10 | 4.7 | 8000 |
| 11 | 0.37 | 13 |
| 12 | 0.35 | 5.1 |
| 13 | 0.56 | 39 |
| 14 | 0.19 | 16 |
| 15 | 0.40 | 34 |
| 16 | 1.3 | 160 |
| 17 | 0.34 | 33 |
| 18 | 5.6 | 190 |
| 1 (hNPW) | 0.36 | 0.043 |

### Industrial Applicability

The compound of the present invention is a ligand selective for GPR7, and is effective for treatment and/or prophylaxis of diseases related to food intake, energy metabolism and endocrine regulation.

## Claims

1. A compound represented by the following formula:
Trp-Tyr-Lys-(X)ₙ-Gly-Arg-Ala-Ala-Gly-Leu-Leu-Ser-Gly-Leu-NH₂ or a pharmaceutically acceptable salt thereof:
wherein:
n is an integer of 2 to 10;
X independently represents
a group represented by -NH-(CH₂)ₘ-CO-,
wherein m is an integer of 2 to 6,
a group represented by the formula: 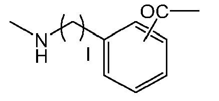 which may have a substituent selected from a substituent group A,
wherein 1 is 0 or 1,
a group represented by the formula: 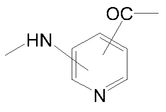 which may have a substituent selected from the substituent group A,
a group represented by the formula: 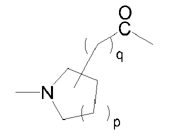 which may have a substituent selected from the substituent group A,
wherein p is an integer of 0 to 2, and q is 0 or 1, or
a group represented by the formula: 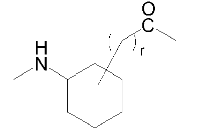 which may have a substituent selected from the substituent group A,
wherein r is 0 or 1;
and the substituent group A consists of a halogen, a methyl group, an ethyl group, a hydroxyl group, a methoxy group, and a nitro group.

2. The compound or the pharmaceutically acceptable salt thereof according to claim 1,
wherein X is a group represented by -NH-(CH₂)₄-CO-.

3. The compound or the pharmaceutically acceptable salt thereof according to claim 2,
wherein n is an integer of 2 to 5.

4. The compound or the pharmaceutically acceptable salt thereof according to claim 2,
wherein n is 3.

5. The compound or the pharmaceutically acceptable salt thereof according to claim 1,
wherein n is 3, two of X's are each a group represented by -NH-(CH₂)₄-CO-, remaining X is a group represented by the formula: 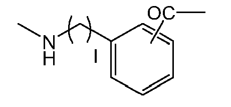 which may have a substituent selected from a substituent group A,
where 1 is 0 or 1,
a group represented by the formula: 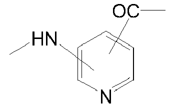 which may have a substituent selected from the substituent group A,
a group represented by the formula: 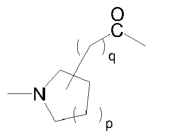 which may have a substituent selected from the substituent group A,
where p is an integer of 0 to 2, and q is 0 or 1, or
a group represented by the formula: 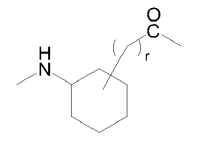 which may have a substituent selected from the substituent group A,
where r is 0 or 1;
and the substituent group A consists of a halogen, a methyl group, an ethyl group, a hydroxyl group, a methoxy group, and a nitro group.

6. The compound or the pharmaceutically acceptable salt thereof according to claim 1,
wherein n is 3, two of X's are each a group represented by -NH-(CH₂)₄-CO-, and remaining X is a group represented by the formula 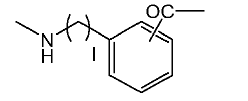 which may have a substituent selected from the group consisting of a halogen, a methyl group, an ethyl group, a hydroxyl group, a methoxy group, and a nitro group,
wherein 1 is 0 or 1.

7. The compound or the pharmaceutically acceptable salt thereof according to claim 1,
wherein n is 3, two of X's are each a group represented by -NH-(CH₂)₄-CO-, and remaining X is a group represented by the formula:

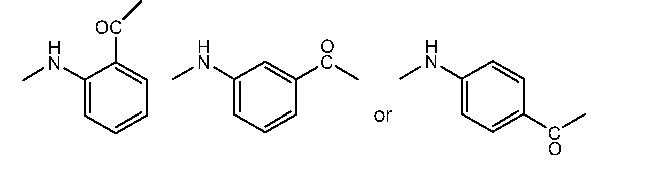

8. A GPR7-selective ligand comprising the compound or the pharmaceutically acceptable salt thereof according to any one of claims 1 to 7.

9. A GPR7-selective agonist comprising the compound or the pharmaceutically acceptable salt thereof according to any one of claims 1 to 7.

10. A drug containing the compound or the pharmaceutically acceptable salt thereof according to any one of claims 1 to 7 as an active ingredient.

11. A prophylactic and/or therapeutic agent for obesity, diabetes or hyperphagia, containing the compound or the pharmaceutically acceptable salt thereof according to any one of claims 1 to 7 as an active ingredient.

12. An aperitive agent containing the compound or the pharmaceutically acceptable salt thereof according to any one of claims 1 to 7 as an active ingredient.

13. A prophylactic and/or therapeutic agent for pituitary hormone secretion insufficiency, containing the compound or the pharmaceutically acceptable salt thereof according to any one of claims 1 to 7 as an active ingredient.

14. A prophylactic and/or therapeutic agent for sleep disorder, containing the compound or the pharmaceutically acceptable salt thereof according to any one of claims 1 to 7 as an active ingredient.

15. An analgesic containing the compound or the pharmaceutically acceptable salt thereof according to any one of claims 1 to 7 as an active ingredient.

16. A prophylactic and/or therapeutic agent for stress, containing the compound or the pharmaceutically acceptable salt thereof according to any one of claims 1 to 7 as an active ingredient.

17. A method for screening compounds binding to GPR7, comprising the steps of:
(a) bringing GPR7 and the compound or the pharmaceutically acceptable salt thereof according to any one of claims 1 to 7 into contact with each other under conditions where a candidate compound is present and under conditions where the candidate compound is not present, and measuring an amount of binding between GPR7 and the compound or the pharmaceutically acceptable salt thereof according to any one of claims 1 to 7, and
(b) selecting the candidate compound which changes the amount of binding between GPR7 and the compound or the pharmaceutically acceptable salt thereof according to any one of claims 1 to 7.
